# FASCICULE DE BREVET EUROPEEN

(11) **EP 0 391 780 B1**
(45) Date de publication et mention de la délivrance du brevet: **04.08.1993**
(21) Numéro de dépôt: 90400894.3
(22) Date de dépôt: 02.04.1990
(51) Int. Cl.: A61K 7/48, A61K 7/06, A61K 37/22

(54) **Composition anti-démangeaisons**
Mittel gegen Juckreiz
Anti-itching composition

(30) Priorité: 05.04.1989 FR 8904464
(43) Date de publication de la demande: 10.10.1990
(73) Titulaire: LABORATOIRES CARILENE, SA, 78360 Montesson (Les Yvelines) (FR)
(72) Inventeur: Desjonqueres, Stéphane, F-78360 Montesson (Les Yvelines) (FR)
(74) Mandataire: Cabinet HERRBURGER

(56) Documents cités:
- EP-A- 0 117 962
- EP-A- 0 225 831
- FR-A- 2 591 104
- FR-A- 2 591 113
- FR-M- 2 330

## Description

La présente invention concerne une composition anti-démangeaisons.

Les démangeaisons cutanées, ou prurit, qui provoquent le besoin de grattage, constituent une sensation courante extrêmement désagréable ; celles-ci peuvent être dues à une maladie dermatologique ou être sans cause apparente précise.

Ces symptômes, extrêmement gênants, constituent la cause de nombreuses consultations en dermatologie ; une localisation très fréquente se situe au niveau du cuir chevelu, ces démangeaisons peuvent être provoquées par des affections cutanées classiques, telles que par exemple la dermite séborrhéique ou le pityriasis capitis, c'est-à-dire les pellicules, ou être consécutives à des réactions allergiques aux produits cosmétiques et colorants utilisés couramment par les coiffeurs.

On a déjà proposé des produits susceptibles d'agir sur les différentes affections pouvant être causes de prurit, mais, les seuls produits de première intention destinés à soulager les démangeaisons sont des compositions à base de cortisone destinées à être appliquées par voie locale qui présentent l'inconvénient de ne pas toujours donner de résultats satisfaisants et, même parfois, de favoriser des phénomènes de rebond, c'est-à-dire d'entraîner des symptômes plus violents que la maladie initiale.

De plus, comme tous les produits à base de cortisone ou de ses dérivés, ces compositions sont relativement toxiques et passent dans le système sanguin des personnes traitées en déséquilibrant le système hormonal traditionnel et en créant une cortico-dépendance entraînant des modalités de sevrage contraignantes. Ces inconvénients limitent en fait l'utilisation de ces compositions à base de cortisone à des cas très sévères, entraînant une gêne considérable dans la vie quotidienne.

La présente invention se propose de remédier à ces inconvénients en proposant une composition anti-démangeaisons dont l'efficacité est au moins égale à celle des compositions classiques à base de cortisone et qui est totalement exempte de toxicité et peut donc être utilisée de manière continue ou prolongée sans risque d'intoxication ou d'effets secondaires indésirables.

A cet effet, l'invention concerne une composition anti-démangeaisons caractérisée en ce qu'elle renferme, en tant que composant actif, au moins un glycérol oxydo ester ayant été préparé par saturation en oxygène et exposition intensive et contrôlée aux ultraviolets, de façon à présenter un taux de peroxydation compris entre 30 et 300 exprimé en milli-équivalents et une teneur en glycérides oxydés comprise entre 5 et 40, la définition du principe actif en spectrophotométrie UV étant de 1 à 6 pour le facteur E 270 et de 8 à 60 pour le facteur E 232 (NF T 60 223).

Ces glycérol oxydo esters présentent la propriété remarquable d'être totalement dénués de présence de radicaux libres cytotoxiques qui sont généralement associés à tout corps oxydé ; cette absence permet de justifier l'emploi de ces composés dans le domaine de la dermatologie ; en effet, l'agressivité des radicaux libres sur la peau est bien connue.

Ces dernières années, on s'est aperçu que les glycérol oxydo esters du type susmentionné pouvaient être utilisés pour le traitement de certaines affections, notamment en rhumatologie ou traumatologie (document FR-A-2 591 108), en tant que compositions cicatrisantes (document FR-A-2 591 112) ou encore pour le traitement de l'herpès (document EP-A-0 225 831). Ces glycérol oxydo esters ont également déjà été utilisés dans le domaine de la cosmétique (document FR-A-2 591 104) ou pour la préparation de produits de massage à usage local plus spécialement destinés aux sportifs (document FR-A-2 591 113).

Par ailleurs, antérieurement aux documents susmentionnés, on avait déjà proposé conformément au document FR-M-2 330 d'utiliser dans des applications thérapeutiques (dermatoses, brûlures, lèpre, toxémies, tuberculose, silicose et états asthéniques) des peroxydes de corps gras éthyléniques à 12 à 20 atomes de carbone.

On peut également mentionner l'existence du document EP-A-0 117 962 qui décrit un procédé de préparation de corps gras peroxydés par transformation biologique à faible énergie ; les corps gras obtenus suite à la mise en oeuvre de ce procédé se sont révélés être des agents de croissance et présentent des propriétés antalgiques, anti-inflammatoires et bactériostatiques.

Indépendamment de ce qui précède, on a découvert, de manière surprenante, que les glycérol oxydo esters conformes à l'invention étaient également susceptibles d'être utilisés pour le traitement des démangeaisons ; l'expérience a en effet montré que, dans la plupart des cas, ils permettent d'obtenir un soulagement rapide, sans avoir à redouter d'effets secondaires nocifs, étant donné qu'il s'agit là d'un produit totalement naturel dépourvu d'une quelconque toxicité.

La composition conforme à l'invention peut, en fonction du type de traitement auquel elle est destinée être présentée sous des formes galéniques quelconques : crèmes, lotions, pommades, gels, pansements imprégnés ou toute formulation galénique adaptée.

Une crème dont le contenu en principe actif peut aller de 2 à 50 % s'est, par exemple, montrée particulièrement efficace pour soulager les prurits de toute nature.

Le produit conforme à l'invention s'est avéré particulièrement efficace pour le traitement des démangeaisons localisées au niveau du cuir chevelu.

Dans ce cas, il est présenté sous forme d'une lotion capillaire conditionnée, de préférence, dans des bouteilles en verre munies d'un applicateur. Le produit doit être appliqué directement sur le cuir chevelu, dans des sillons définis en écartant les cheveux aux endroits où les démangeaisons sont les plus fortes ; le soulagement est généralement obtenu au bout de 5 à 10 minutes, voire instantanément, après un un léger massage destiné à permettre l'introduction du produit dans le derme.

La lotion capillaire conforme à l'invention s'est avérée particulièrement intéressante pour le traitement d'intolérances cutanées légères, voire sévères, mais passagères consécutives à certains soins des cheveux effectués par les coiffeurs, notamment pour les sensations d'irritation et de brûlures du cuir chevelu ainsi que des plaques irritatives rosées ou rouges apparaissant fréquemment à la lisière de la plantation des cheveux (front, oreilles, nuque ...) à la suite de colorations, permanentes ...

Conformément à l'invention, le glycérol oxydo ester mis en oeuvre peut être quelconque pour peu qu'il satisfasse à la définition susmentionné : l'expérience a montré que les produits préférentiels sont obtenus à partir d'huile végétales naturelles.

La quantité de composants actifs mis en oeuvre est, de préférence, comprise entre 2 et 5 % en poids pour la lotion.

A titre d'exemple, on a pu obtenir des résultats particulièrement satisfaisants en utilisant une lotion capillaire contenant, approximativement en pourcentages pondéraux :
- 3 % d'huile d'amande douce peroxydée,
- 13 % d'émulsifiants,
- 23 % d'éthanol à 99°
- 0,1 % de parfum,
- le complément à 100 % étant constitué par de l'eau déminéralisée.

Comme il a déjà été indiqué, l'invention n'est pas limitée à une lotion capillaire, mais peut également s'appliquer à une crème ou à un gel anti-démangeaisons.

Dans ce cas, le glycérol oxydo-ester, notamment mis en oeuvre à raison de 2 à 50 % en poids, provient de préférence d'une huile végétale naturelle, notamment d'huile d'amande douce, d'huile de noisette, d'huile d'arachide et/ou d'huile de maïs peroxydée.

On a obtenu des résultats particulièrement satisfaisants en mettant en oeuvre une composition contenant approximativement en pourcentages pondéraux, 2 à 50 % d'huile d'amande douce peroxydée, 1 % de parfum et des excipients à usage cosmétique, le complément à 100 % étant constitué par de l'eau déminéralisée.

Il convient de remarquer que la composition conforme à l'invention peut également être utilisée dans le domaine vétérinaire, particulièrement pour le traitement du prurit du chien.

L'activité de la composition et plus particulièrement de la lotion capillaire conforme à l'invention sera mise en lumière grâce à l'exemple ci-dessous qui est un compte-rendu d'une étude multi-centrique effectuée par huit médecins sur 51 patients souffrant de problèmes de démangeaisons soit diffuses soit localisées au niveau du cuir chevelu.

La fréquence de l'apparition des crises allait d'environ une fois par semaine pour quelques patients, à quotidiennement, voire plus, dans la plupart des cas.

68,6 % des patients présentaient une dermatose associée, tandis que 52,9 % avaient déjà été traités antérieurement et localement avec d'autres produits (corticoïdes). La durée moyenne de l'étude a été de 16,97 jours.

Les sujets susmentionnés ont été traités par application quotidienne de la lotion sur les zones atteintes le matin ou le soir à la convenance du patient et à raison d'environ 5 ml de lotion par application.

Il est à noter que pour cette expérimentation, le produit a été appliqué en première intention sans traitement ou décapage préalable des dermites pouvant être présentes (antiseptique, acide salicylé ...) ou traitement à base de corticoïdes des lésions pré-existantes.

Chez 21 sujets, soit 41 % des cas, on a observé une sédation totale de la démangeaison.

Chez 24 sujets, soit 47 % des cas, on a observé une sédation partielle de la démangeaison.

La différence, soit 6 cas, représente les sujets chez lesquels il a été constaté une absence d'activité.

L'activité a été instantanée chez 9 sujets (soit 17,6 % des cas) et précoce, c'est-à-dire au bout d'environ 5 mn chez 21 sujets (soit 41,2 % des cas), ce qui correspond à un résultat instantané ou précoce dans environ 59 % des cas.

On a noté une activité plus tardive inférieure à 21 heures chez 15 sujets, soit 29,4 % des cas.

Cette étude permet donc de conclure à une activité de la lotion conforme à l'invention dans 88,2 % des cas, cette activité se manifestant dans un délai moyen de quelques secondes à moins de 21 heures et à une absence d'activité chez 6 sujets, soit 11,8 % des cas.

Il est à noter que la réapparition des symptômes est survenue au bout de 4 jours en moyenne après arrêt du traitement alors que dans 37 cas sur 51, la fréquence des démangeaisons était quotidienne ou pluri-hebdomadaire.

La tolérance a été jugée excellente chez 32 sujets, soit 65,3 % des cas et bonne chez 15 sujets, soit 30,6 % des cas (deux sujets n'ont pas communiqué les résultats du point de vue de la tolérance).

La lotion capillaire conforme à l'invention a donc montré une tolérance bonne à excellente dans 95,5 % des cas.

La tolérance a été moyenne chez 2 sujets, et 1 sujet a dû interrompre le traitement avec apparition de picotements quelques minutes après l'application de la lotion ; ces picotements ont cessé dès l'arrêt du traitement. On peut penser à une sensibilité individuelle due autant à l'excipient qu'au principe actif.

Par ailleurs, 48 % des sujets ont indiqué ne pas avoir eu les chevaux gras après le traitement, 25 % des sujets ont trouvé que leurs cheveux étaient brillants après le traitement, et 87 % des sujets ont trouvé que leurs cheveux n'étaient pas lourds après ce traitement.

53 % des réponses font ressortir un emploi très agréable ou agréable.

73,5 % des sujets ont trouvé que la lotion conforme à l'invention était plus efficace ou aussi efficace que les traitements locaux habituels (corticoïdes).

Ce compte-rendu montre clairement que l'activité du produit a été positive.

Des essais cliniques ont, par ailleurs, été menés avec la crème sur les prurits atypiques "sine materia" notamment sur la peau sèche et prurigineuse des personnes âgées ave succès.

D'autres essais ont démontré une excellente activité sur les prurits anogénitaux. On peut citer les travaux du Docteur GELINET qui a obtenu, sur 15 manifestations prurigineuses anales, 10 guérisons, 4 améliorations et un état stationnaire. Le Docteur TROCHET, proctologue, a obtenu des résultats comparables.

## Revendications

1. Utilisation pour la fabrication d'une composition anti-démangeaisons d'au moins un glycérol oxydo-ester ayant été préparé par saturation en oxygène et exposition intensive et contrôlée aux ultra-violets, de façon à présenter un taux de peroxydation compris entre 30 et 300 exprimé en milli-équivalents et une teneur en glycérides oxydées comprise entre 5 et 40, ce glycérol oxydo-ester étant essentiellement exempt de radicaux libres cytotoxiques et sa définition en spectrophotométrie UV étant de 1 à 6 pour le facteur E 270 et de 8 à 60 pour le facteur E 232.

2. Utilisation d'au moins un glycérol oxydo ester selon la revendication 1 pour la fabrication d'une lotion capillaire anti-démangeaisons.

3. Utilisation selon la revendication 2 d'huile d'amande douce peroxydée.

4. Lotion capillaire destinée à être utilisée selon l'une quelconque des revendications 2 et 3, caractérisée en ce qu'elle comporte entre 2 et 5 % en poids de glycérol oxydo ester.

5. Lotion capillaire selon la revendication 4, caractérisée en ce qu'elle contient, approximativement en pourcentages pondéraux, 3 % d'huile d'amande douce peroxydée, 13 % d'agents émulsifiants, 23 % d'éthanol à 99° et 0,1 % de parfum, le complément à 100 % étant constitué par de l'eau déminéralisée.

6. Crème ou gel anti-démangeaisons destinée à être utilisée conformément à la revendication 1, caractérisé en ce que le glycérol oxydo ester provient d'une huile végétale naturelle, notamment d'huile d'amande douce, d'huile de noisette, d'huile d'arachide et/ou d'huile de maïs peroxydée.

7. Composition anti-démangeaisons selon la revendication 6, caractérisée en ce qu'elle renferme entre 2 et 50 % en poids de glycérol oxydo ester.

8. Composition anti-démangeaisons selon la revendication 7, caractérisée en ce qu'elle contient approximativement, en pourcentages pondéraux, 2 à 50 % d'huile d'amande douce peroxydée, 1 % de parfum et des excipients à usage cosmétique, le complément à 100 % étant constitué par de l'eau déminéralisée.

## Patentansprüche

1. Verwendung zumindest eines Glycerol-Oxido-Esters zur Herstellung einer Zusammensetzung gegen Juckreiz, hergestellt durch Sauerstoffsättigung und intensive und kontrollierte Utraviolett-Bestrahlung, derart, daß ein Peroxidationsbetrag zwischen 30 und 300, ausgedrückt in Milliäquivalenten, und ein Gehalt an oxidierten Glyceriden zwischen 5 und 40 vorliegen, wobei der Glycerol-Oxido-Ester im wesentlichen frei von cytotoxischen freien Radikalen ist und seine UV-Spektrophotometrie-Definition von 1 bis 6 für den Faktor E 270 und von 8 bis 60 für den Faktor E 232 reicht.

2. Verwendung zumindest eines Glycerol-Oxido-Esters nach Anspruch 1 für die Herstellung einer Kapillarlotion gegen Juckreiz.

3. Verwendung nach Anspruch 2 eines peroxidierten Süßmandelöls.

4. Kapillarlotion, bestimmt zur Verwendung nach einem der Ansprüche 2 und 3, dadurch gekennzeichnet, daß sie zwischen 2 und 5 Gew.-% Glycerol-Oxido-Ester umfaßt.

5. Kapillarlotion nach Anspruch 4, dadurch gekennzeichnet, daß sie angenähert, in Gewichtsprozenten, 3 % peroxidiertes Süßmandelöl, 13 % Emulgiermittel, 23 % Ethanol zu 99° und 0,1 % Duftstoff enthält, wobei die Ergänzung auf 100 % von demineralisiertem Wasser gebildet ist.

6. Creme oder Gel gegen Juckreiz, bestimmt zur Verwendung gemäß Anspruch 1, dadurch gekennzeichnet, daß der Glycerol-Oxido-Ester aus peroxidiertem Süßmandelöl, Haselnußöl, Erdnußöl und/oder Maisöl gewonnen ist.

7. Zusammensetzung gegen Juckreiz nach Anspruch 6, dadurch gekennzeichnet, daß sie zwischen 2 und 50 Gew.-% Glycerol-Oxido-Ester umfaßt.

8. Zusammensetzung gegen Juckreiz nach Anspruch 7, dadurch gekennzeichnet, daß sie angenähert, in Gewichtsprozenten, 2 bis 5 % peroxidiertes Süßmandelöl, 1 % Duftstoff und Bindemittel für kosmetische Anwendung enthält, wobei die Ergänzung auf 100 % von demineralisiertem Wasser gebildet ist.

## Claims

1. Use, for the manufacture of an anti-itching composition, of at least one glycerol oxido ester having been prepared by saturation with oxygen and intensive and controlled exposure to ultraviolet radiation, so as to have a peroxidation level of between 30 and 300 expressed in milliequivalents and a content of oxidised glycerides of between 5 and 40, this glycerol oxido ester being essentially free of cytotoxic free radicals and its definition in UV spectrophotometry being 1 to 6 for factor E 270 and 8 to 60 for factor E 232.

2. Use of at least one glycerol oxido ester according to Claim 1, for the manufacture of an anti-itching hair lotion.

3. Use according to Claim 2 of a peroxidised expressed almond oil.

4. Hair lotion intended to be used according to either of Claims 2 and 3, characterised in that it contains between 2 and 5% by weight of glycerol oxido ester.

5. Hair lotion according to Claim 4, characterised in that it contains approximately, in per cent by weight, 3% of peroxidised expressed almond oil, 13% of emulsifying agents, 23% of ethanol at 99° and 0.1% of perfume, the balance for 100% consisting of demineralised water.

6. Anti-itching cream or gel intended to be used in conformity with Claim 1, characterised in that the glycerol oxido ester is derived from a natural vegetable oil, especially peroxidised expressed almond oil, hazelnut oil, groundnut oil and/or maize oil.

7. Anti-itching composition according to Claim 6, characterised in that it contains between 2 and 50% by weight of glycerol oxido ester.

8. Anti-itching composition according to Claim 7, characterised in that it contains approximately, in per cent by weight, 2 to 50% of peroxidised expressed almond oil, 1% of perfume and excipients for cosmetic use, the balance for 100% consisting of demineralised water.
